# EUROPEAN PATENT APPLICATION

(11) **EP 1 516 597 A1**
(43) Date of publication of application: **23.03.2005**
(21) Application number: 03739968.0
(22) Date of filing: 25.06.2003
(51) Int. Cl.: A61F 2/06

(54) **DRUG ELUTING STENT**

(30) Priority: 27.06.2002 CN 02112242; 24.10.2002 CN 02146905; 17.12.2002 CN 02155138; 28.02.2003 CN 03115596; 28.03.2003 CN 03116063; 28.05.2003 CN 03128906
(71) Applicant: Microport Medical (Shanghai) Co., Ltd., 201203 Shanghai (CN)
(72) Inventor: ZHANG, Yi, Pudong New District, 201203 Shanghai (CN); WANG, Jianke, Pudong New District, 201203 Shanghai (CN); TANG, Zhirong, Pudong New District, 201203 Shanghai (CN); HE, Shuiying, Pudong New District, 201203 Shanghai (CN); ZHOU, Shunhua, Pudong New District, 201203 Shanghai (CN); GAO, Runlin, Pudong New District, 201203 Shanghai (CN); LI, Junfei, Pudong New District, 201203 Shanghai (CN); ZHANG, Ying, Pudong New District, 201203 Shanghai (CN); LUO, Qiyi, Pudong New District, 201203 Shanghai (CN)
(74) Representative: Kritzenberger & Zeuner
(86) International application number: PCT/CN2003/000489
(87) International publication number: WO 2004/002367

(57) **Abstract**

The invention relates to a drug eluting stent and a method for making the stent. The stent comprises a stent body and coats covering on the stent body, it is **characterized in that**: 2-4 coats are provided on the surface of the stent body, wherein at least two coats are drug coats. The drug coats comprise 0.5-99% polymer by weight, 0-10% additive by weight and 0.5-99% active ingredient by weight. A primer layer is provided between the drug coats and the stent body. A compact control-releasing layer is also coated on the surface of the drug coats.

## Description

### Field of the Inventions

The inventions described below relate the field of medical devices, and provides a new drug-eluting stent that includes multi-layer coatings.

### Background of the Inventions

Since Sigwart implanted the first stent in a coronary artery in 1986, stents have developed into the primary treatment for occlusive blood vessel disease. Now, eighty percent of atherosclerotic lesions are treated with stent placement.

Percutaneous Transluminal Coronary Angioplasty (PTCA), in which a balloon is used to open obstructed arteries, has been widely used to treat atherosclerotic lesions. However, this technique is limited by the vexing problems of re-occlusion and restenosis. Restenosis results from the excessive proliferation of smooth muscle cell (SMC), and the rate of restenosis is above 20%. Thus about one in five patients treated with PTCA must be treated again within several months.

In our Chinese patent application 02112242.3, we have disclosed an intravascular stent with active therapeutic agents to prevent restenosis and reduce the need for subsequent retreatment after PTCA.

### Summary

This invention is based on Chinese patent application 02112242.3, and is intended to provided anti-stenotic stent coatings which are more uniform, more securely adherent to stent, more resistant to cracking and flaking, and, thus, to prevent multiple complications after stenting. The drug eluting stent comprises a stent coated with a primer, one or more drug-eluting layers, and a barrier layer. Therapeutic compounds such as immunosuppressants are dispersed in a polymer matrix that is applied to the stent, over the primer, and a barrier layer comprising parylene or other suitable polymer.

### Brief Description of the Drawings

Fig. 1 shows the sustained release effect of parylene coatings of different molecular weight.
Fig. 2 shows the release rate of drugs using parylene coatings of different thickness.
Fig. 3 shows the result of in-vitro dilation without base coating.
Fig. 4 shows the result of in-vitro dilation with base coating.

### Detailed Description of the Inventions

The new stent is composed of metal stent and a coating on the stent surface. The stent provides an expandable substrate adapted for implantation in vessel of a human body, for a multi-layer coating that includes a drug eluting layer, a primer layer, and a barrier coating applied as a topcoat. The coating has 1, 2, 3 or 4 layers, with one or more layers containing a drug or therapeutic compound. The drug eluting layer comprises a therapeutic compound (for example an immunosuppressant compound) which is dispersed within a polymeric matrix. The drug-layer contains 0.5-99% polymer, 0-10% additive and 0.5-99% active materials.

To enhance the adhesion of the drug-layer to the metal stent, a primer or base coating is applied to the stent. The base coating may be composed of one or several kinds of polymer, including polyvinyl alcohol or polybutylmethacrylate (PBMA).

On the surface of the drug-layer, there may be an additional surface layer, containing 0.5-99% polymer, 0-10% additive and 0-99% active materials.

A rapid release of therapeutic agents from a,drug-eluting stent may delay the biological process of endothelialization. To avoid this phenomenon, this stent may include a compact barrier coating. Material of this coating may be parylene and its derivatives, PTFE, etc. Parylene is a highly pure, chemically inert coating material. It is highly biocompatible. The US FDA has approved the use of parylene in human implants. Parylene coatings can enhance biocompatibility and surface smoothness of medical instruments. In order to improve the haemo-compatibility, some anti-platelet agents (Cilostazol, Plavix, Ticlid and etc) may be added to the compact coating, which thickness change from 0.01-20microns.

The active agents of surface layer and drug layer may be the same or different than the active agents in the main coating. And the surface layer can carry a litter drug or blank. As a release barrier, it can adjust the release rate to meet the particular needs of particular applications, and prevent acute or sub-acute thrombus.

The thickness of the entire coating, as contemplated for this invention, is 0.1 to 100 microns.

The active agents (therapy drug or gene) in the stent coating maybe chosen from the following: immunosuppressant compounds, anti-thrombogenic agents, anti-cancer agents, hormones, and other anti-stenosis drugs. Suitable immunosuppressants include ciclosporinA (CsA), FK506, DSG(15-deoxyspergualin, 15-dos), MMF, rapamycin and its derivatives, CCI-779, FR 900520, FR 900523, NK86-1086, daclizumab, depsidomycin, kanglemycin-C, spergualin, prodigiosin25-c, cammunomicin, demethomycin, tetranactln, tranilast, stevastelins, myriocin, gllooxin, FR 651814, SDZ214-104, bredinin, WS9482, and steroid. Suitable anti-thrombogenic drugs include heparin, aspirin, hirudin and etc., GPIIb/IIIa receptor inhibitor as tirofiban, eptifibatide, cilostazol, plavix, Ticlid and etc. Suitable anti-cancer agents include methotrexate, purine, pyridine, and botanical (e.g. paclitaxel, colchicines and triptolide), epothilone, antibiotics, and antibody. Suitable additional anti-stenosis agents include batimastat, NO donor, 2-chlorodeoxyadenosine, 2-deoxycoformycin, FTY720, Myfortic, ISA (TX) 247, AGI-1096, OKT3, Medimmune, ATG, Zenapax, Simulect, DAB486-IL-2, Anti-ICAM-1, Thymoglobulin, Everolimus, Neoral, Azathipprine (AZA), Cyclophosphamide, Methotrexate, Brequinar Sodium, Leflunomide, Mizoribine; Gene therapy formulations including: Keratin 8, VEGF, and EGF, PTEN, Pro-UK, NOS, or C-myc may also be used.

The methods of preventing restenosis includes inhibiting VSMC hyperplasia or migration, promoting endothelial cell growth, or inhibiting cell matrix proliferation with the delivery of suitable compounds from the drug-eluting layers.

The polymer used to form the coating may be polyester (lactide, glyatide, and -caprolactone), cellules, poly(vinyl alcohol), PMMA, PBMA, povidone, poly(ethylene-co-vinyl alcohol), arabia rubber, bassora gum, EVAC, cellulose or various other suitable compounds.

Suitable additives to the polymer coating include cross-linking agents, dispersants (wetting agents) and plasticizers.

The function of the cross linking agents is to provide structural integrity to the coating, and cross-linking agents such as acylamine, amidoformate may be used. The function of the dispersants (wetting agents) is to enhance dispersion of the polymer, to make the distribution of components of the solution more uniform, and ionic or non-ionic surfactants are suitable. The function of the plasticizer is to improve the mechanical characteristics of the coating. Plasticizers including linear polymers such as polyaether may be used.

The present invention provides preparation methods of drug-eluting stents as follows:

1 part drug and 2-1000 parts solvent are put into a container and dispersed. Stents are coated uniformly with the dispersed solution and then cured in a vacuum oven for 0.5-72 hours at 20-200°C. This process can be repeated with the same drug, or a different drug, dispersed in solution.

Thereafter the stents are coated with 1,4-dimethylbenzene through vacuum vapor deposition.

The solvents used in the present invention are able to disperse polymers, active components and additives uniformly. The solvents should be stable, non-reactive with the polymers, active components and additives. The solvents should not affect on the therapeutic effect of active components; and The solvents should be volatile and readily evaporate from the coating while the coating is curing.

These solvents include water; alcohol and ketone such as glycerin, isopropanol acetone, cyclohexanone butanone, ester such as ethyl acetate, butyl acetate, alkane such as n-hexane chloroform dichloromethane aromatic hydrocarbon such as benzene, methylbenzene; heterocyclic aromatic hydrocarbon such as tetrahydrofuran; amide such as N,N-dimethylformamide and N,N-dimethylacetamide.

The polymers, active components, and additives may be dispersed by stirring or ultrasonic emulsification. Thereafter, the coating may be applied to the stent by dipping, spray coating, or a combination of both. The coatings may be cured by heat or radiation.

The coating method of the present invention may be used for making stents intended for use in treatment of coronary vessels and cerebral and peripheral vessel obstructions. The stents may be balloon expandable stents or self-expanding stents of the type currently used in the treatment of coronary arteries, cerebral arteries, carotid arteries, pulmonary arteries, kidney arteries, and other vessel obstructions.

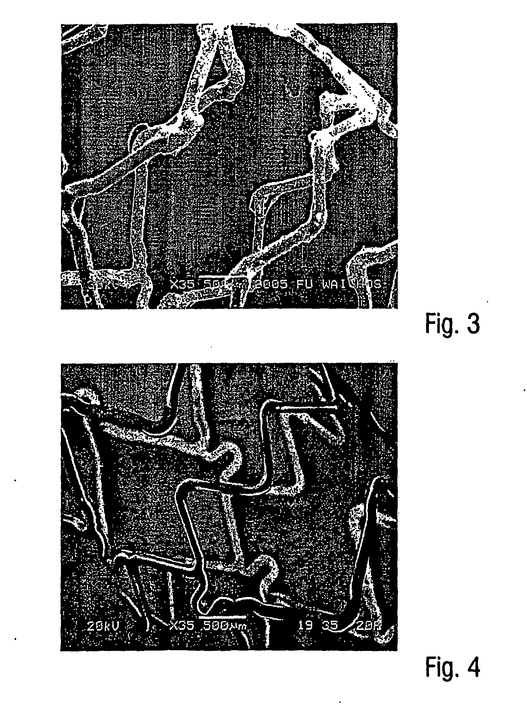

The coatings are uniformly deposited on the stent surface, without breakage. The coatings remain in there original condition in blood at 37°C as well as after dilation. As seen in the comparison of Figure 3, which is a stent coated with a drug eluting compound without first applying a primer to the stent, with Figure 4, which is a stent made according to the procedures described above. The drug eluting coating of the unprimed stent of Figure 3 exhibits cracking and flaking of the coating, while the drug eluting coating of the primed stent of Figure 4 remains intact after dilation. The active components are able to avoid complication and have the ability to treat local pathological changes and lesions.

The following examples illustrate the application of the inventions described above to various embodiments of drug-eluting stents.

### Example 1

Application of the primer (base coating) of the stent: 0.5g copolymer of ethylene and vinyl alcohol is put into 10ml N, N-dimethylacetamide. The mixture is dispersed at 80°C and then sprayed onto stents. Thereafter the stents are dried in a vacuum oven for 2 hours at 120°C.

### Example 2

Barrier layer-preparation of parylene coating:

The present invention provides parylene and its derivatives as release control materials. Parylene is prepared by vacuum vapor deposition of 1,4-dimethylbenzene. First, 1-4-dimethylbenzene is heated to 950°C to form dimethylbenzene dimer which cracks into monomer vapor at 680°C later. Then steel stents are put in a deposition chamber at room temperature. Monomer vapor is introduced in the deposition chamber to form compact polymer coatings on the surface of stents. The molecular weight of polymer is estimated at 500,000.

### Example 3

Preparation of barrier layer which has an antiplatelet-aggregation function.

The decomposition process for obtaining the parylene monomer is as same as example 2. While the monomer steam is introduced into the substrate deposition chamber, the platelet antagonist grains (such as Cilostazol, Ticlid, Plavix and so on) are introduced into the deposition chamber. As a result, an even, compact, controllable release layer with antiplatelet aggregation function can be formed on the surface of the substrate.

### Example 4

0.2 g poly (buthyl methacrylate) is put into 10ml mixed solvent (N, N-dimethylacetamide/ethyl acetate = 1:4), and is dispersed (or dissolved) at room temperature. 0.2g Rapamycin is then added to the solution and dispersed at room temperature. Then the mixture is sprayed onto the surface of the stents, which have been prepared as described in EXAMPLE 1. Thereafter the stents are solidified in a vacuum oven for 2 hours at 40°C. Then the controllable release layer with the thickness of 0.2 microns is prepared as described in EXAMPLE 2.

### Example 5

0.1g ethylene-vinyl acetate copolymer and 0.2 g poly (buthyl methacrylate) are added in 10ml iso-propylalcohol and dispersed (or dissolved) at room temperature. Then 0.2 g colchicine is put into the solution and dispersed at room temperature. Then the mixture is sprayed onto the surface of the stents, which have been prepared as described in EXAMPLE 1. Thereafter the stents are solidified in a vacuum oven for 2 hours at 80°C. Then the controllable release layer with the thickness of 0.5 microns is prepared as described in EXAMPLE 3.

### Example 6

0.3g lactide-ε-caprolactone copolymer is put into 5ml chloroform and dispersed (or dissolved) at room temperature. Then 0.1 g actinomycin is added to the solution and dispersed at room temperature. Then the stents is immersed in the solution for 1 to 30 min, and are solidified in a vacuum oven for 2 hours at 60°C. Thereafter the controllable release layer with the thickness of 0.05 microns is prepared as described in EXAMPLE 3.

### Example 7

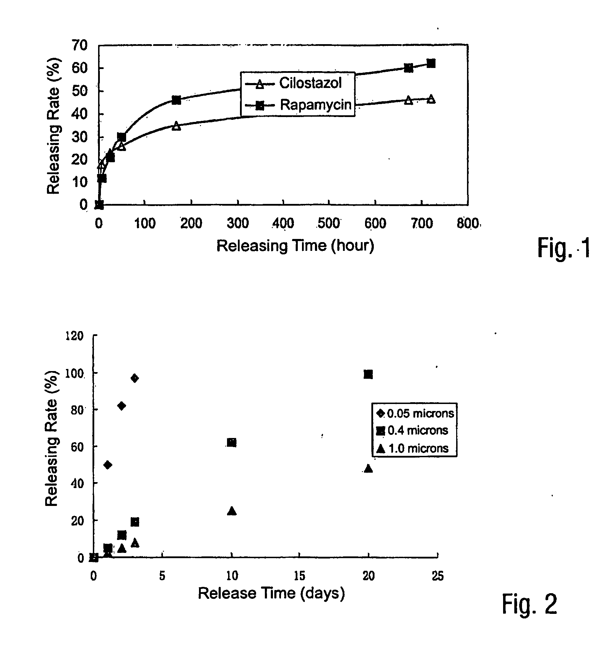

One part polyactic acid and one part polycaprolactone are dissolved in 100 parts chloroform. Then one-part micro molecule drugs (such as Cilostazol) as well as one-part macromolecule drugs (such as Rapamycin) are dispersed at room temperature. Then the mixture is sprayed onto the surface of the stents, and the stents are dried in a vacuum oven at 30°C. The parylene-coated layer is prepared as described in EXAMPLE 2. The release effect of the parylene-coated layer on the different molecular weight drugs is shown as Figure 1. It shows that the different molecular weight drugs will have different release rate. While the molecular weight is larger, the release rate will be slower. And the increase of the coating layer's thickness will result in the slowdown of the release rate.

### Example 8

One part poly (buthyl methacrylate) and one part ethylene-vinyl acetate copolymer are dissolved in 100 parts tetrahydrofuran. Then one-part micro molecule drugs (such as 10-camptothecin) are dispersed at room temperature. Then the mixture is sprayed onto the surface of the stents that have been prepared as described in EXAMPLE 1. And the stents are dried in a vacuum oven at 30°C. Thereafter the parylene-coated layer is prepared as described in EXAMPLE 2. The release effect of the parylene-coated layer with the different coating thickness (such as 0.05 microns, 0.1 microns, 0.2 microns, 0.4 microns and 0.5 microns) on the micro molecular drugs is shown as Figure 2. It shows that the parylene-coated layer can control the release rate of the micro molecule drugs. The thicker layer will result in the slower release rate. So varying the thickness of the parylene-coated layer can vary the release rate.

### Example 9

Two part lactide-caprolactone copolymer is dissolved in 100 part chloroform. Then two part micro molecule drugs (such as FTY720) are dispersed at room temperature. Then the mixture is sprayed onto the surface of the stents that have been prepared as described in EXAMPLE 1 and the stents are dried in a vacuum oven at 30°C. Thereafter the parylene-coated layer is prepared as described in EXAMPLE 2. The result of the release studies in vitro shows that the release rate can be well controlled and the object of the long-term drug release can be achieved.

### Example 10

Two parts poly (butyl methacrylate) is dissolved in 100 parts normal ethyl butyrate. Then one-part micro molecule drugs (such as Epo-D) are dispersed at room temperature. Then the mixture is sprayed onto the surface of the stents that have been prepared as described in EXAMPLE 1 and the stents are dried in a vacuum oven at 30°C. Thereafter the parylene-coated layer is prepared as described in EXAMPLE 2. The result shows that the parylene-coated layer can well control the release rate of the micro molecule drugs. The release time can be varied from 1 day to 90 day.

### Example 11

Two part lactide-ε-caprolactone copolymer is dissolved in 100 part chloroform. Then two part micro molecule drugs (such as Triperygium Wilfordii) are put in the mixture. Then the mixture is sprayed onto the surface of the stents that have been prepared as described in EXAMPLE 1. And the stents are dried in a vacuum oven at 30°C. Thereafter the parylene-coated layer is prepared as described in EXAMPLE 2. The result of the release studies in vitro shows that the release rate can be well controlled and the object of the long-term drug release can be achieved.

When the encephalic stents are implanted in some animal models that have glioma, they can restrain glioma effectively until it disappears.

### Example 12

0.20 g poly (buthyl methacrylate) and 0.20g ethylene-vinyl acetate copolymer are put into 10ml chloroform. Then 0.1g Paclitaxel and 0.2g Cilostazol are added to the mixture and dispersed at room temperature. Then the mixture is sprayed onto the surface of the stents, which have been prepared as described in EXAMPLE 1. Thereafter the stents are solidified in air for 2 hours. Then the above process will be repeated till the amount of coated drug researches 300µg/cm2. Then the stents are dried in a vacuum oven. Thereafter the parylene-coated layer is prepared as described in EXAMPLE 2, and the thickness is 0.1 microns.

### Example 13

0.20 g poly (buthyl methacrylate) and 0.20g ethylene-vinyl acetate copolymer are put into 10ml chloroform. Then 0.1g Paclitaxel is added to the mixture and dispersed at room temperature. Then the mixture is sprayed onto the surface of the stents, which have been prepared as described in EXAMPLE 1. Thereafter the stents are solidified in air for 2 hours. Then the above process will be repeated till the amount of coated drug researches 100µg/cm2. Then the stents are dried in a vacuum oven. Thereafter the parylene and Cilostazol mixed layer is coated as described in EXAMPLE 3, and the thickness is 0.5 microns.

### Example 14

0.20 g poly (buthyl methacrylate) is put into 10ml N, N-dimethylacetamide. Then 0.18g Epo-D is added to the mixture and dispersed at room temperature. Then the mixture is sprayed onto the surface of the stents, which have been prepared as described in EXAMPLE 1. Thereafter the stents are solidified in a vacuum oven for 24 hours at 40°C. Thereafter the parylene and Ticlid mixed layer is coated as described in EXAMPLE 3, and the thickness is 0.3 microns.

### Example 15

Dipping the drug eluting stent in 37°C physiological saline for 10 minutes, and dilated with balloon, then scanned with SEM.

Fig. 3 shows the stent without base coating, and the coating peeled off after dilation.

Fig. 4 shows the stent with base coating, and the coating without crack after dilation.

Thus, while the preferred embodiments of the devices and methods have been described in reference to the environment in which they were developed, they are merely illustrative of the principles of the inventions. Other embodiments and configurations may be devised without departing from the spirit of the inventions and the scope of the appended claims.

## Claims

1. The new drug eluting stent with multiplayer coatings, and the character of the coating including that the coating has 1-4 layers, at least two layers containing drug. The drug-layer contains 0.5-99% polymer, 0-10% additive and 0.5-99% active materials.

2. The drug eluting stent of claim 1 wherein there is a base coating between drug layer and metal stent, and the base coating is composed of one or more polymers.

3. The drug eluting stent of claim 2 wherein there may be a surface layer upon the drug layer, which composed of 0.5-99%(wt) polymer, 0-10% additives and 0-99% active agents.

4. The drug eluting stent of claim 2 wherein there is a compact barrier, which composed of parylene and its derivative or PTFE.

5. The drug eluting stent of claim 4 wherein the barrier layer contains anti-thrombus agents.

6. The drug eluting stent of claim 4 wherein the thickness of barrier layer range from 0.01 to 20 microns.

7. The drug eluting stent of claim 1 wherein there is a compact layer upon the coating, which composed of parylene and its derivative or PTFE.

8. The drug eluting stent of claim 7 wherein the barrier layer contains anti-thrombus agents.

9. The drug eluting stent of claim 7 wherein the thickness of barrier layer range from 0.01 to 20 microns.

10. The drug eluting stent of claim 2 wherein the active agents of surface layer maybe the same of drug layer or not.

11. The drug eluting stent of claim 1 wherein the thickness of each coating maybe 0.1-100 microns.

12. The drug eluting stent of claim 1 to 11, wherein the active agent of drug layer chosen from one or multiple therapy drug/gene, which including Anti-thrombus agents, anti-tumor, microbe immunosuppressant, hormone and other anti-stenosis agent.

13. The drug eluting stent of claim 12 wherein the anti-thrombus agent chosen from heparin, aspirin, hirudin, colchicine and GPIIb/IIIa receptor inhibitor.

14. The drug eluting stent of claim 12 wherein the GPIIb/IIIa receptor inhibitor including tirofiban, eptifibatide, cilostazol, plavix, Ticlid and etc.

15. The drug eluting stent of claim 12 wherein the anticancer agents chosen from Methotrexate, purine, pyridine, and botanical (e.g. paclitaxel, colchicines and triptolide), epothilone, antibiotics, and antibody.

16. The drug eluting stent of claim 15 wherein the antibiotic is actinomycin-D and the botanical is paclitaxel or EPO-D.

17. The drug eluting stent of claim 12 wherein the immunosuppressant including ciclosporinA (CsA), FK506, DSG(15-deoxyspergualin, 15-dos), MMF, Rapamycin and derivative, CCI-779, FR 900520, FR 900523, NK86-1086, daclizumab, depsidomycin, kanglemycin-C, spergualin, prodigiosin25-c, cammunomicin, demethomycin, tetranactln, tranilast, stevastelins, myriocin, gllooxin, FR 651814, SDZ214-104, bredinin, WS9482, and steroid.

18. The drug eluting stent of claim 12 wherein other anti-stenosis agents including: batimastat, NO donor, 2-chlorodeoxyadenosine, 2-deoxycoformycin, FTY720, Myfortic, ISA (TX) 247, AGI-1096, OKT3, Medimmune, ATG, Zenapax, Simulect, DAB486-IL-2, Anti-ICAM-1, Thymoglobulin, Everolimus, Neoral, Azathipprine (AZA), Cyclophosphamide, Methotrexate, Brequinar Sodium, Leflunomide, Mizoribine.

19. The drug eluting stent of claim 18 wherein metallicprotein inhibitor is MMP inhibitor.

20. The drug eluting stent of claim 12 wherein the therapy gene including: Keratin 8, VEGF, and EGF, PTEN, Pro-UK, NOS, C-myc.

21. The drug eluting stent of claim 12 wherein the polymer applied in this invention including polyester (lactide, glyatide, and ε-caprolactone), cellules, poly(vinyl alcohol), PMMA, PBMA, povidone, poly(ethylene-co-vinyl alcohol), arabia rubber, bassora gum, EVAC, cellulose and the compound of all of above.

22. The drug eluting stent of claim 12 wherein the additives including cross-linking agents, dispersant, Plasticizer.

23. The drug eluting stent of claim 22 wherein the cross-linking agent including acylamine, amidoformate, acid or alkali.

24. The drug eluting stent of claim 22 wherein the dispersant is anion or non-ion surfactants.

25. The drug eluting stent of claim 22 wherein the plasticizer is linear polymer.

26. The drug eluting stent of claim 1-25 wherein the preparation methods including: dissolving the drug in solvent, and coating the solution on the surface of stent then dried and cured.

27. The drug eluting stent of claim 26 wherein the coating of parylene by vapor deposit polymerization.

28. The drug eluting stent of claim 26 wherein the solvents including water; alcohol and ketone such as glycerin, isopropanol acetone, cyclohexanone butanone, ester such as ethyl acetate, butyl acetate, alkane such as n-hexane chloroform dichloromethane aromatic hydrocarbon such as benzene, methylbenzene; heterocyclic aromatic hydrocarbon such as tetrahydrofuran; amide such as N,N-dimethylformamide, N,N-dimethylacetamide.

29. The drug eluting stent of claim 1-25 wherein may be apply in the interventional therapy of the cardiovascular, nerve and peri-vessel block.

30. The drug eluting stent of claim 1-25 wherein the stent including balloon expanded or self-expanded stent.
